(19) Europäisches Patentamt
European Patent Office
Office européen des brevets

(11) Numéro de publication: 0 376 848 A1

(12) **DEMANDE DE BREVET EUROPEEN**

(21) Numéro de dépôt: **89403667.2**

(22) Date de dépôt: **28.12.89**

(51) Int. Cl.5: **C07D 413/04, C07D 417/04, A61K 31/44**

Une requête en rectification, conformément à la règle 88 CBE: page 3 des revendications où la numérotation des revendications 13 à 17 a été revue, a été présentée. Il est statué sur cette requête au cours de la procédure engagée devant la division d'examen (Directives relatives à l'examen pratiqué à l'OEB, A-V, 2.2).

(30) Priorité: **28.12.88 IT 2312188**

(43) Date de publication de la demande: **04.07.90 Bulletin 90/27**

(84) Etats contractants désignés: **CH DE FR GB IT LI NL**

(71) Demandeur: **ROUSSEL-UCLAF**
**35, boulevard des Invalides**
**F-75007 Paris(FR)**

(72) Inventeur: **Toja, Emilio**
**Via Piezzo 80**
**Milano(IT)**
Inventeur: **Bonetti, Carla**
**Via Beccalino**
**Fontanella (Bergamo)(IT)**
Inventeur: **Barzaghi, Fernando**
**Via Monteverdi 21**
**I-20052 Monza Milan(IT)**
Inventeur: **Galliani, Giulio**
**13, Via Silva**
**1 Monza(IT)**

(74) Mandataire: **Vieillefosse, Jean-Claude et al**
**Roussel-Uclaf 111, route de Noisy B.P. 9**
**F-93230 Romainville(FR)**

(54) **Dérivés de la 1,2,5,6-tétrahydropyridine substitués par un radical thiazolyle ou oxazolyle, leur procédé et les intermédiaires de préparation, leur application comme médicaments et les compositions les renfermant.**

(57) L'invention concerne les composés de formule générale (I) :

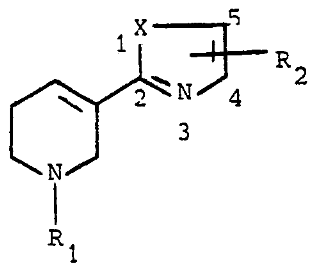

(I)

dans laquelle
- Y est oxygène ou soufre,
- $R_1$ est hydrogène, alkyle ($C_{1-8}$), alkényle ou alkynyle ($C_{1-6}$), hydroxyle, $OCOalc_1$, $OCONHalc_2$ dans lesquels $alc_1$ ou $alc_2$ représente alkyle ($C_{1-6}$), $CO_2alc_3$ dans lequel $alc_3$ représente alkyle ou alkényle ($C_{1-6}$), $Oalc_4$ dans lequel $alc_4$ représente alkyle ($C_{1-6}$) , $OCOAr_1$, $OCONHAr_2$ ou $CO_2Ar_3$ dans lequels $Ar_1$, $Ar_2$ ou $Ar_3$ représente aryle ($C_{6-14}$),
-$R_2$ est hydrogène, alkyle ($C_{1-6}$), alkényle ou alkynyle ($C_{1-6}$), alkoxyalkyle $alc'_1Oalc'_2$ dans lequel $alc'_1$ et

EP 0 376 848 A1

alc$'_2$ sont alkyles ($C_{1-6}$), hydroxyalkyle dans lequel alkyle ($C_{1-6}$), aryle ($C_{6-14}$),
ainsi que leurs sels avec les acides, leur procédé de préparation, leur application comme médicaments et les compositions les renfermant.

**Nouveaux dérivés de la 1,2,5,6-tétrahydropyridine substitués par un radical thiazolyle ou oxazolyle. leur procédé et les intermédiaires de préparation, leur application comme médicaments et les compositions les renfermant.**

La présente invention concerne de nouveaux dérivés de la 1,2,5,6-tétrahydropyridine substitués par un radical thiazolyle ou oxazolyle, leur procédé et les intermédiaires de préparation, leur application comme médicament et les compositions les renfermant.

L'invention a pour objet les composés de formule générale (I) :

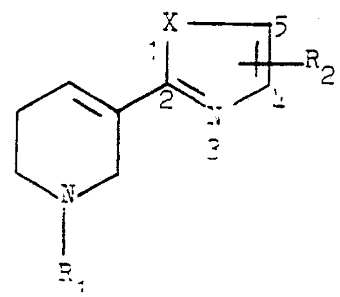

(I)

dans laquelle

- X représente un atome d'oxygène ou de soufre,
- $R_1$ représente un atome d'hydrogène, un radical alkyle linéaire, ramifié ou cyclisé renfermant jusqu'à 8 atomes de carbone, un radical alkényle ou alkynyle renfermant jusqu'à 6 atomes de carbone, un radical hydroxyle, un radical $OCOalc_1$ dans lequel $alc_1$ représente un radical alkyle renfermant jusqu'à 6 atomes de carbone, un radical $OCONHalc_2$ dans lequel $alc_2$ représente un radical alkyle renfermant jusqu'à 6 atomes de carbone, un radical $CO_2alc_3$ dans lequel $alc_3$ représente un radical alkyle ou alkényle renfermant jusqu'à 6 atomes de carbone, un radical $Oalc_4$ dans lequel $alc_4$ représente un radical alkyle renfermant jusqu'à 6 atomes de carbone, un radical $OCOAR_1$, $OCONHAr_2$ ou $CO_2Ar_3$ dans lesquels $Ar_1$, $Ar_2$ ou $Ar_3$ représentent un radical aryle renfermant jusqu'à 14 atomes de carbone,
- $R_2$ représente un atome d'hydrogène, un radical alkyle linéaire, ramifié ou cyclisé, renfermant jusqu'à 6 atomes de carbone, un radical alkényle ou alkynyle renfermant jusqu'à 6 atomes de carbone, un radical alkoxyalkyle $alc'_1$ $Oalc'_2$ dans lequel $alc'_1$ et $alc'_2$ représentent des radicaux alkyles renfermant jusqu'à 6 atomes de carbone, un radical hydroxyalkyle dans lequel le radical alkyle renferme jusqu'à 6 atomes de carbone ou un radical aryle renfermant jusqu'à 14 atomes de carbone, ainsi que leurs sels d'addition avec les acides organiques ou minéraux.

Parmi les sels d'addition avec les acides, on peut citer ceux formés avec les acides minéraux, tels que les acides chlornydrique, bromhydrique, sulfurique ou phosphorique ou avec les acides organiques comme l'acide formique, acétique, propionique, benzoïque, maléïque, fumarique, succinique, tartrique, citrique, oxalique, glyoxylique, aspartique, alcanesulfoniques, tels que les acides méthane ou éthane sulfoniques, arylsulfoniques tels que les acides benzène ou paratoluènesulfoniques.

Dans la définition des composés de formule générale (I), lorsque l'un des substituants représente un radical alkyle, il s'agit de préférence d'un radical méthyle, éthyle, n-propyle, isopropyle, n-butyle, sec-butyle, terbutyle ou isobutyle. Lorsque l'un des substituants représente un radical alkényle ou alkynyle, il s'agit de préférence d'un radical vinyle, allyle, éthynyle ou propynyle.

L'invention a notamment pour objet les composés dans lesquels $R_1$ représente un radical hydroxy ainsi que leurs sels d'addition avec les acides minéraux ou organiques, ceux dans lesquels $R_1$ représente un atome d'hydrogène ainsi que leurs sels d'addition avec les acides organiques ou minéraux ainsi que ceux dans lesquels $R_1$ représente un radical alkyle linéaire, ramifié ou cyclique comme par exemple un radical méthyle ainsi que leurs sels d'addition avec les acides organiques ou minéraux.

Parmi les composés préférés de l'invention, on peut citer les composés dans lesquels X représente un atome d'oxygène ainsi que leurs sels d'addition avec les acides organiques ou minéraux.

Parmi les composés préférés de l'invention, on peut également citer les composés dans lesquels $R_2$ représente un atome d'hydrogène et ceux dans lesquels $R_2$ représente un radical alkyle linéaire, ramifié ou cyclique renfermant jusqu'à 6 atomes de carbone comme par exemple un radical méthyle ainsi que leurs sels d'addition avec les acides organiques ou minéraux.

Parmi les composés préférés de l'invention, on peut citer les composés dans lesquels X représente un

atome de soufre et $R_2$ représente un radical alkyle, linéaire, ramifié ou cyclique comme par exemple un radical méthyle ainsi que leurs sels d'addition avec les acides organiques ou minéraux.

L'invention a particulièrement pour objet les composés dont la préparation est donnée ci-après dans la partie expérimentale et tout particulièrement :

- la 1-hydroxy 3-(oxazol-2-yl) 1,2,5,6-tétrahydropyridine,
- la 3-(oxazol-2-yl) 1,2,5,6-tétrahydropyridine,
- la 3-(4-méthyloxazol-2-yl) 1,2,5,6-tétrahydropyridine,
- la 3-(5-méthyloxazol-2-yl) 1,2,5,6-tétrahydropyridine,

et leurs sels d'addition avec les acides organiques ou minéraux.

Les composés de l'invention présentent de très intéressantes propriétés pharmacologiques et notamment une activité cholinomimétique centrale par voie orale importante de longue durée d'action.

L'invention a donc pour objet les produits de l'invention en tant que médicaments, utiles notamment dans le traitement de la maladie d'Alzheimer ou de la démence sénile et également dans le traitement des troubles de la mémoire.

L'invention a plus particulièrement pour objet en tant que médicaments, les composés des exemples 2, 6, 8 et 10.

La posologie usuelle est variable selon l'affection en cause, le sujet traité et la voie d'administration, elle peut être comprise entre 1 mg et 100 mg/jour, par exemple, entre 1 et 15 mg/jour en une ou plusieurs prises pour le produit des exemples 6 ou 10 administré par voie orale.

La présente invention a également pour objet les compositions pharmaceutiques renfermant comme principe actif au moins un produit de formule (I). Les compositions pharmaceutiques de l'invention peuvent être solides ou liquides et se présenter sous les formes pharmaceutiques couramment utilisées en médecine humaine, comme, par exemple, les comprimés simples et dragéifiés, les gélules, les granulés, les suppositoires, les préparations injectables ; elles sont préparées selon les méthodes usuelles.

Le ou les principes actifs peuvent y être incorporés à des excipients habituellement employés dans ces compositions pharmaceutiques, tels que le talc, la gomme arabique, le lactose, l'amidon, le stéarate de magnésium, le beurre de cacao, les véhicules aqueux ou non, les corps gras d'origine animale ou végétale, les dérivés paraffiniques, les glycols, les divers agents mouillants, dispersants ou émulsifiants, les conservateurs.

L'invention a également pour objet un procédé, caractérisé en ce que l'on soumet un composé de formule (II) :

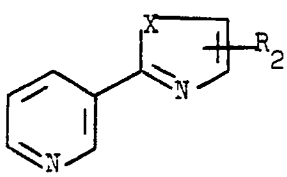

(II)

dans laquelle $R_2$ et X conservent leur signification précédente à l'action d'un composé de formule (III) :

$R'_1$-Hal (III)

dans laquelle $R'_1$ peut prendre toutes les valeurs données ci-dessus pour $R_1$ à l'exception de l'hydrogène, pour obtenir le composé de formule (IV) :

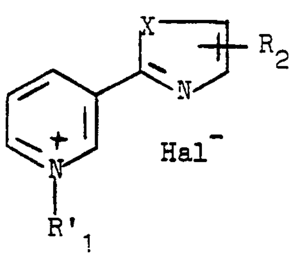

(IV)

que l'on soumet à l'action d'un agent de réduction pour obtenir le composé de formule ($I_A$) :

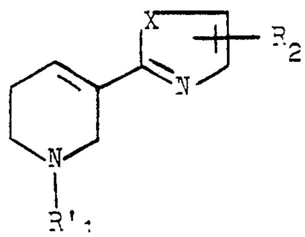

$(I_A)$

que l'on salifie si désiré ou que l'on soumet le cas échéant à l'action d'un agent de clivage du groupe $R'_1$ pour obtenir le composé de formule ($I_B$) :

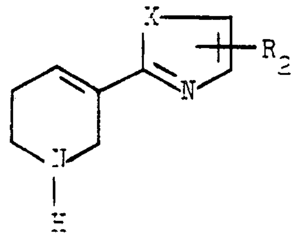

$(I_B)$

que l'on salifie si désiré ou que l'on soumet, le cas échéant, à l'action d'un agent d'oxydation pour obtenir un composé de formule (I) dans laquelle $R_1$ représente un groupe $OR''_1$, dans lequel $R''_1$ représente un groupement protecteur du radical hydroxy que l'on salifie si désiré ou si désiré élimine le groupement protecteur pour obtenir un composé de formule (I) dans laquelle $R_1$ représente un radical hydroxy libre que, si désiré, l'on salifie.

Dans un mode de réalisation préféré du procédé de l'invention,
- le composé de formule (III) utilisé est un iodure,
- l'agent de réduction est le borohydrure de sodium,
- l'agent de clivage du groupement $R'_1$ est le chloroformiate d'alpha-chloroéthyle,
- l'agent d'oxydation est par exemple un peroxyde comme le peroxyde de benzoyle ou le peroxide de bis (diphénylphosphinyl),
- l'élimination du groupement protecteur du radical hydroxy se fait de manière usuelle. On utilise par exemple un métal alcalin tel que le sodium au sein d'un alcool inférieur tel que le méthanol ou l'éthanol ou l'acide sulfurique 1N dans les mêmes solvants.

Le composé de formule (II) dans laquelle X est un atome de soufre et $R_2$ un radical méthyle en position 4 est connu, il peut être préparé selon le procédé de préparation de la <3-(4-méthyl thiazol-2-yl) pyridine décrit dans Helv. Chim. Acta. 28, 820 (1945).

Les composés de formule (II) dans lesquels X est un atome de soufre, portant un substituant en position 4 peuvent être préparés selon le schéma réactionnel :

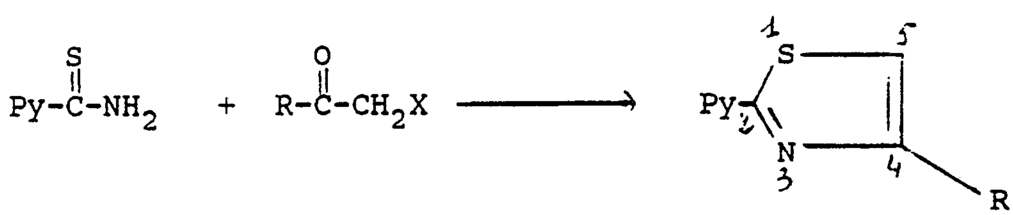

Py = 3-pyridyl ; $X_1$ = Cl, Br.

Les composés de formule (II) dans lesquels X est un atome d'oxygène, portant un substituant en position 4 peuvent être préparés selon le schéma réactionnel :

Py = 3-pyridyl

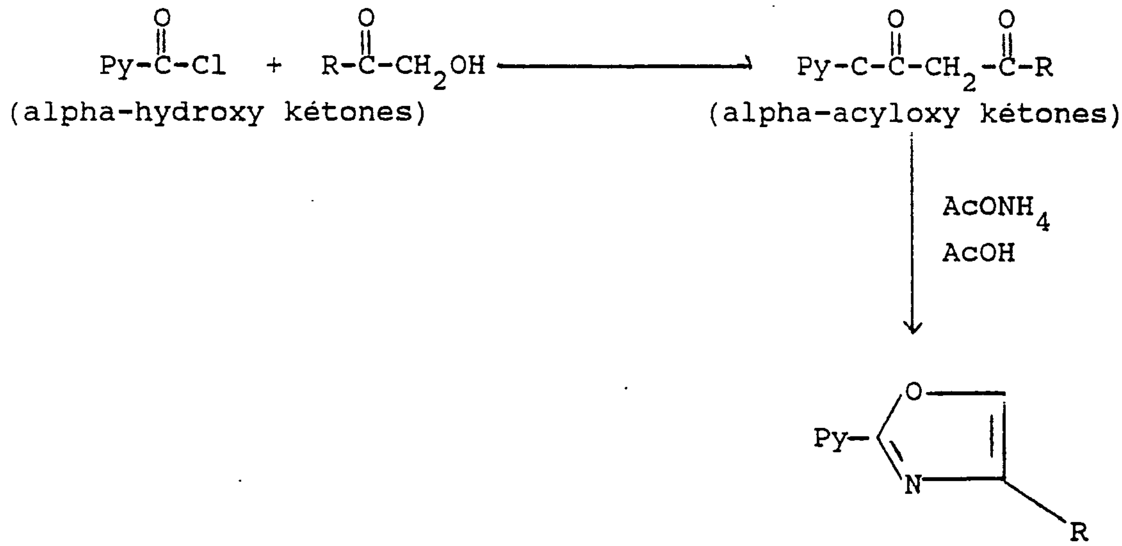


Le composé de formule (II) dans laquelle X est un atome d'oxygène et $R_2$ un radical méthyle en position 4 est un produit nouveau et est en lui- même un objet de la présente invention.

Il peut être préparé selon le procédé décrit ci-dessus ou encore selon le procédé décrit ci-après dans la partie expérimentale qui peut être résumé comme suit :

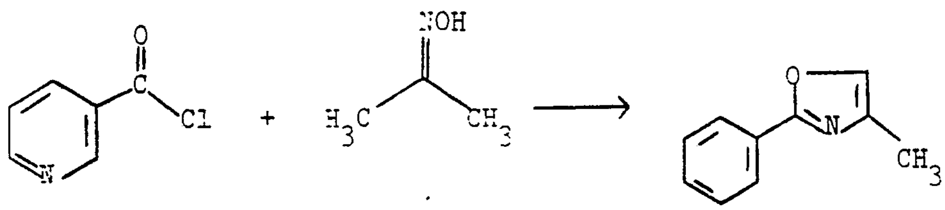

Les composés de formule (II) dans lesquels X est un atome d'oxygène, portant un substituant en position 5 peuvent être préparés selon le schéma réactionnel :

Py = 3-pyridyl ; $X_1$ = Cl, Br.

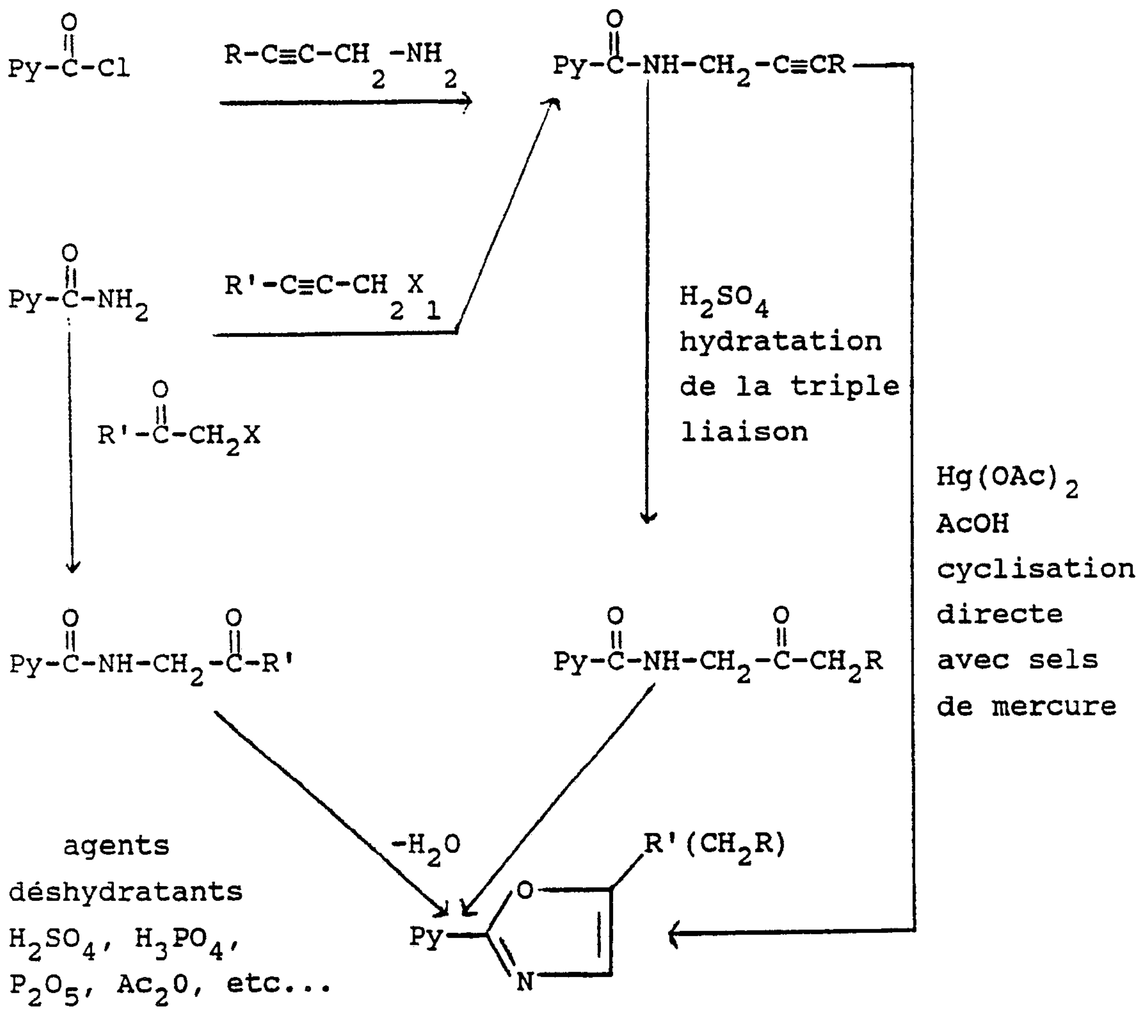


Les exemples suivants illustrent l'invention sans toutefois la limiter.

**Exemple 1 : 1-méthyl 3-(4-méthyloxazol-2-yl) 1,2,5,6-tétrahydropyridine et son chlorhydrate.**

**Stade A :** Iodure de 1-méthyl 3-(4-méthyloxazol-2-yl) pyridinium.

On mélange 10,8 g de 3-(4-méthyloxazol-2-yl) pyridine et 19,4 g d'iodure de méthyle dans 140 $cm_3$ d'éthanol puis chauffe 6 heures au reflux. On évapore le solvant, cristallise le résidu dans un mélange d'éthanol et d'éther et obtient 17 g de produit attendu que l'on recristallise dans l'éthanol. F = 203 °C.

| Analyse : $C_{10}H_{11}IN_2O$ | | | |
|---|---|---|---|
| Calculé :<br>Trouvé : | C% 39,76<br>39,47 | H% 3,67<br>3,74 | N% 9,27<br>9,15 |

**Stade B :** 1-méthyl 3-(4-méthyloxazol-2-yl) 1,2,5,6-tétrahydropyridine et son chlorhydrate.

On ajoute à 0° C 4,7 g de borohydrure de sodium à 17 g de produit obtenu au stade A dans 200 cm3 de méthanol. On laisse revenir à température ambiante et agite 3 heures. On évapore le solvant, reprend dans l'eau, extrait à l'acétate d'éthyle, purifie par chromatographie sur silice (éluant : chloroforme-méthanol 9-1), distille à 110° C sous 0,05 mbar et recueille 5,3 g de produit, sous forme de base, auquel on ajoute 96 $cm_3$ d'une solution aqueuse d'acide chlorhydrique 0,1N. On concentre à sec, cristallise dans le mélange éthanol-éther et obtient 1,9 g de chlorhydrate attendu. F = 228-230° C.

| Analyse : $C_{10}H_{14}N_2O$, HCl | | | |
|---|---|---|---|
| Calculé :<br>Trouvé : | C% 55,94<br>55,79 | H% 7,04<br>7,18 | N% 13,05<br>12,97 |

**Préparation 1 : [3-(4-méthyloxazol-2-yl) pyridine].**

Dans 5,1 g d'acétoxime refroidi, on ajoute 30 g de chlorure de nicotinoyl, puis chauffe le mélange à 130° C. On maintient sous agitation le milieu réactionnel exothermique pendant 6 heures à 140° C. On refroidit, ajoute de l'eau glacée, alcalinise avec du carbonate de sodium et extrait au chlorure de méthylène. On évapore le solvant, reprend le résidu à l'éther, filtre, évapore le solvant et distille à 150° C sous 0,08 mbar. On obtient 3,5 g de produit attendu que l'on purifie par chromatographie sur silice (éluant : cyclo hexane-acétate d'éthyle 7-3) puis nouvelle distillation. Après refroidissement, on obtient un produit solide. F = 40° C.

| Analyse : $C_9H_8N_2O$ | | | |
|---|---|---|---|
| Calculé :<br>Trouvé : | C% 67,49<br>67,13 | H% 5,03<br>5,12 | N% 17,49<br>17,26 |

**Exemple 2 : Chlorhydrate de 3-(4-méthyloxazol-2-yl) 1,2 ,5,6-tetrahydropyridine.**

A une solution de 3,65 g de [1-méthyl 3-(4-méthyloxazol-2-yl) 1,2,5,6-tétrahydropyridine] dans 70 $cm_3$ de dichloroéthane, on ajoute à -5° C sous atmosphère inerte, 3,25 g de chloroformiate d'alpha-chloroéthyle et chauffe 2 heures au reflux. On évapore le solvant, reprend le résidu dans l'éther, filtre, évapore le solvant, reprend le résidu dans 30 $cm_3$ de méthanol, chauffe au reflux pendant 45 minutes, élimine le solvant et obtient, après cristallisation dans l'éthanol, 1,9 g de produit attendu. F = 276° C (décomp.).

| Analyse : $C_9H_{12}N_2O$, HCl | | | |
|---|---|---|---|
| Calculé :<br>Trouvé : | C% 53,87<br>53,61 | H% 6,53<br>6,66 | N% 13,96<br>13,76 |

**Exemple 3 : 1-méthyl 3-(4-méthylthiazol-2-yl) 1,2,5,6-tétrahydropyridine et son chlorhydrate.**

**Stade A :** Iodure de 1-méthyl 3-(4-méthylthiazol-2-yl) pyridinium.

On abandonne à l'obscurité pendant 10 jours, 8,3 g de[3-(4-méthylthiazol-2-yl) pyridine] et 4,5 $cm_3$ d'iodométhane dans 30 $cm_3$ d'éthanol. On filtre et obtient 14,6 g de produit que l'on recristallise dans l'éthanol. F = 184-186° C.

| Analyse : $C_{10}H_{11}IN_2S$ | | | |
|---|---|---|---|
| Calculé :<br>Trouvé : | C% 37,75<br>37,49 | H% 3,49<br>3,43 | N% 8,80<br>8,71 |

**Stade B** : 1-méthyl 3-(4-méthylthiazol-2-yl) 1,2,5,6-tétrahydropyridine et son chlorhydrate.

On ajoute en maintenant la température inférieure à 10 °C, 2,57 g de borohydrure de sodium à 14,4 g de produit obtenu au stade A dans 100 $cm_3$ de méthanol. On agite 2 heures à température ambiante, concentre à sec, reprend le résidu dans l'eau, extrait à l'acétate d'éthyle, sèche et évapore le solvant. On chromatographie le résidu sur silice (éluant : chlorure de méthylène-méthanol 9-1), distille à 140 °C sous 0,007-0,008 mbar et recueille 5,5 g de produit attendu sous forme de base. On dissout 1,57 g de base dans 5 $cm_3$ d'éthanol, ajoute 80,8 $cm_3$ d'acide chlorhydrique 0,1N, concentre à sec, et cristallise dans l'isopropanol. On obtient 1,79 g de chlorhydrate attendu. F = 199-202 °C (décomp.).

| Analyse : $C_{10}H_{14}N_2S$, HCl | | | |
|---|---|---|---|
| Calculé :<br>Trouvé : | C% 52,05<br>52,18 | H% 6,12<br>6,23 | N% 12,14<br>12,06 |

**Exemple 4 : Chlorhydrate de 3-(4-méthylthiazol-2-yl) 1,2 ,5,6-tétrahydropyridine.**

On dissout 3,64 g de [1-méthyl 3-(4-méthylthiazol-2-yl) 1,2,5,6-tétrahydropyridine] dans 70 $cm_3$ de dichloroéthane, ajoute à 0 °C sous atmosphère inerte 2,95 g de chloroformiate d'alpha-chloroéthyle, chauffe au reflux pendant 1 heure et demie, élimine le solvant, reprend le résidu à l'éther et filtre. On concentre le filtrat à sec, reprend le résidu dans 40 $cm_3$ de méthanol et chauffe 30 minutes au reflux. On élimine le solvant, cristallise 2 fois dans l'éthanol et l'éther et obtient 2,67 g de produit attendu. F = 184-185 °C (décomp.).

| Analyse : $C_9H_{12}N_2S$, HCl | | | |
|---|---|---|---|
| Calculé :<br>Trouvé : | C% 49,88<br>49,71 | H% 6,05<br>5,97 | N% 12,93<br>13,04 |

**Exemple 5 : 1-méthyl 3-(oxazol-2-yl) 1,2,5,6-tétrahydropyridine et son chlorhydrate.**

**Stade A** : Iodure de 1-méthyl 3-(oxazol-2-yl) pyridinium.

On chauffe 2 heures au reflux 11,3 g de [(3-oxazol-2-yl) pyridine] préparée comme indiqué dans Helv. Chim. Acta 45 375 (1962) et 22,4 g d'iodure de méthyle dans 150 $cm_3$ d'éthanol puis abandonne le milieu réactionnel pendant 2 jours. On évapore le solvant, cristallise dans l'éthanol 95° et obtient 17 g de produit. F = 241 °C (décomp.) après recristallisation dans l'éthanol.

| Analyse : $C_9H_9IN_2O$ | | | |
|---|---|---|---|
| Calculé :<br>Trouvé : | C% 37,52<br>37,29 | H% 3,15<br>3,12 | N% 9,72<br>9,56 |

**Stade B** : 1-méthyl 3-(oxazol-2-yl) 1,2,5,6-tétrahydropyridine et son chlorhydrate.

On ajoute à 0 °C 4,85 g de borohydrure de sodium à 16,7 g de produit préparé au stade A dans 200 $cm_3$ de méthanol. On poursuit la réaction à température ambiante pendant 1 heure, évapore à sec, reprend le résidu dans l'eau, extrait à l'acétate d'éthyle, sèche et concentre à sec. On purifie par chromatographie sur silice (éluant : chloroforme-méthanol 9-1). On distille à 120 °C sous 0,06 mbar et obtient 5,8 g de produit attendu sous forme de base. On ajoute 110 $cm_3$ d'acide chlorhydrique 0,1N à 1,5 g de base obtenue ci-dessus, concentre à sec, cristallise dans l'éthanol et l'éther et recueille 1,45 g de chlorhydrate attendu. F = 202-204 °C.

| Analyse : $C_9H_{12}N_2O$, HCl | | | |
|---|---|---|---|
| Calculé :<br>Trouvé : | C% 53,87<br>53,64 | H% 6,53<br>6,47 | N% 13,96<br>13,79 |

**Exemple 6 : Chlorhydrate de 3-(oxazol-2-yl) 1,2,5,6-tétrahydropyridine**

On refroidit à -5 °C sous atmosphère inerte 4 g de 1-méthyl 3-(oxazol-2-yl) 1,2,5,6-tétrahydropyridine dans 80 $cm_3$ de dichloroéthane et ajoute 3,88 g de chloroformiate d'alphachloroéthyle. On chauffe au reflux 1 heure et demie, évapore, reprend dans le diéthyléther, filtre, concentre la phase éthérée, reprend le résidu dans 30 $cm_3$ de méthanol, chauffe 40 minutes à l'ébullition, évapore à sec et obtient après cristallisation dans l'éthanol et l'éther, 2,1 g de produit attendu. F = 188-191 °C.

| Analyse : $C_8H_{10}N_2O$, HCl | | | |
|---|---|---|---|
| Calculé :<br>Trouvé : | C% 51,48<br>51,65 | H% 5,94<br>5,87 | N% 15,01<br>15,24 |

**Exemple 7 : 1-méthyl 3-(5-méthyloxazol-2-yl) 1,2,5,6-tétrahydropyridine et son chlorhydrate**

**Stade A** : Iodure de 1-méthyl 3-(5-méthyloxazol-2-yl) pyridinium.

On dissout 5 g de 3-(5-méthyloxazol-2-yl) pyridine préparé comme indiqué dans Chimica Therap 4, 437 (1973) dans 50 $cm_3$ d'éthanol, ajoute 3 $cm_3$ d'iodométhane et chauffe au reflux pendant 10 heures. On refroidit, décante, ajoute de l'éther, filtre et obtient 8,68 g de produit. F = 147-148 °C après cristallisation dans l'éthanol.

| Analyse : $C_{10}H_{11}N_2OI$ | | | |
|---|---|---|---|
| Calculé :<br>Trouvé : | C% 39,76<br>39,54 | H% 3,67<br>3,75 | N% 9,27<br>9,24 |

**Stade B : 1-méthyl 3-(5-méthyloxazol-2-yl) 1,2,5,6-tétrahydropyridine et son chlorhydrate.**

On ajoute à -5°/-3° C 1,6 g de borohydrure de sodium à 8,48 g de produit obtenu au stade A dans 110 $cm_3$ de méthanol. On poursuit la réaction en agitant 2 heures à température ambiante, élimine le solvant, reprend dans l'eau le résidu et extrait à l'acétate d'éthyle. On sèche et évapore le solvant. Après chromatographie sur silice (éluant : chloroforme-méthanol 9-1), on distille à 125-130° C sous 0,07 mbar et obtient 3,29 g de produit attendu sous forme de base. On ajoute 64,5 $cm_3$ d'acide chlorhydrique 0,1N à 1,15 g de la base obtenue ci-dessus, concentre à sec, cristallise dans le méthanol et l'éther et recueille 1,24 g de chlorhydrate attendu. F = 203-205° C (décomp.).

| Analyse : $C_{10}H_{14}N_2O$, HCl | | | |
|---|---|---|---|
| Calculé :<br>Trouvé : | C% 55,94<br>55,71 | H% 7,04<br>7,22 | N% 13,05<br>13,01 |

**Exemple 8 : Chlorhydrate de 3-(5-méthyloxazol-2-yl) 1,2,5,6-tétrahydropyridine.**

On dissout sous atmosphère inerte, 2,1 g de 1-méthyl 3-(5-méthyloxazol-2-yl) 1,2,5,6-tétrahydropyridine préparée comme à l'exemple 7 dans 25 cm3 de dichloroéthane et ajoute à 0° C 1,4 $cm_3$ de chloroformiate d'alpha-chloroéthyle. On chauffe au reflus pendant 2 heures, élimine le solvant, reprend le résidu à l'éther, filtre, concentre à sec le filtrat, reprend le résidu dans 20 $cm_3$ de méthanol et chauffe de nouveau au reflux pendant 40 minutes, concentre à sec et recueille après cristallisation dans l'isopropanol et l'éther, 2,06 g de produit attendu que l'on recristallise dans l'isopropanol/acétone. On obtient 1,42 g de produit fondant à 182-184° C.

| Analyse : $C_9H_{12}N_2O$, HCl | | | |
|---|---|---|---|
| Calculé :<br>Trouvé : | C% 53,87<br>54,05 | H% 6,53<br>6,54 | N% 13,96<br>14,07 |

**Exemple 9 : 1-benzoyloxy 3-(oxazol-2-yl) 1,2,5,6-tétrahydropyridine.**

On ajoute 2,5 g de chlorhydrate de 3-(oxazol-2-yl) 1,2,5,6-tétrahydropyridine préparé comme à l'exemple 6 et 3,22 g de carbonate de potassium dans une solution comprenant 4,1 g de peroxyde de benzoyle dans 30 $cm_3$ de chloroforme. Après 2 heures d'agitation à température ambiante, on filtre, évapore à sec, purifie par chromatographie sur silice (éluant : toluène-acétate d'éthyle 6-4). On obtient 2,4 g de produit que l'on cristallise dans le cyclohexane. F = 90-92° C.

| Analyse : $C_{15}H_{14}N_2O_3$ | | | |
|---|---|---|---|
| Calculé : | C% 66,66 | H% 5,22 | N% 10,36 |
| Trouvé : | 66,86 | 5,30 | 10,24 |

**Exemple 10 : 1-hydroxy 3-(oxazol-2-yl) 1,2,5,6-tétrahydropyridine et son chlorhydrate.**

On ajoute 5,4 g de produit préparé comme à l'exemple 6 dans 150 $cm_3$ d'éther à une solution comprenant 0,65 g de sodium, 100 $cm_3$ de méthanol. Après 1 heure de réaction à température ambiante, on évapore les solvants, reprend le résidu dans l'acide chlorhydrique dilué, extrait à l'éther, alcalinise la phase acide à l'aide de bicarbonate de soude puis extrait à l'acétate d'éthyle. On purifie par chromatographie sur alumine (éluant : chloroforme-méthanol 9-3). On obtient 3 g de produit attendu sous forme de base. On ajoute 110 $cm_3$ d'acide chlorhydrique 0,1N à 1,83 g de la base préparée ci-dessus, concentre à sec et récupère après cristallisation dans l'éthanol et l'éther 1,3 g de chlorhydrate attendu cristallisé F = 158-160 °C et 0,7 g de chlorhydrate non cristallisable.

| Analyse : $C_8H_{10}N_2O_2$, HCl | | | |
|---|---|---|---|
| Calculé : | C% 47,42 | H% 5,47 | N% 13,82 |
| Trouvé : | 47,48 | 5,54 | 13,73 |

**Exemple 11 :** Composition pharmaceutique.

On a préparé des comprimés répondant à la formule suivante :

| | |
|---|---|
| - Produit de l'exemple 6 ..... | 50 mg |
| - Excipient q.s. pour un comprimé ..... | 300 mg |
| (Détail de l'excipient : lactose, talc, amidon traité, amidon de riz, stéarate de magnésium). | |

**Exemple 12 :** Composition pharmaceutique.

On a préparé des gélules répondant à la formule suivante :

| | |
|---|---|
| - Produit de l'exemple 10 ..... | 60 mg |
| - Excipient q.s. pour un gélule ..... | 300 mg |
| (Détail de l'excipient : talc, stéarate de magnésium, aérosil). | |

## ETUDE PHARMACOLOGIQUE

**Toxicité aigüe.**

L'essai est réalisé sur des souris mâles ($CD_1$ Charles Rivers) de 22 à 24 g, à jeun depuis 16 heures. On administre les produits par voie orale à la dose de 1000, 500, 250, 125, 62 et 31 mg/kg. On note la mortalité pendant les 7 jours suivant le traitement.

| Exemple | $DL_{50}$ en mg/kg |
|---|---|
| 2 | 125 |
| 6 | 200 |
| 8 | 500 |
| 10 | 300 |
| Aérocoline, HBr | 600 |

**Test de l'iléon isolé de cobaye.**

On prélève des fragments d'iléon de cobayes tués par décapitation. L'iléon isolé est placé dans 10 $cm_3$ de solution de Tyrode à 37° C et aéré par un mélange d'oxygène (95%) et de gaz carbonique (5%). Les contractions dues aux produits sont enregistrées à l'aide d'un capteur relié à un polygraphe. Les produits à tester sont ajoutés, aux concentrations comprises entre $1.10^{-3}$M et $1.10^{-8}$M/l.

Les produits présentant un effet contracturant sont testés vis à vis de l'atropine et de l'hexaméthonium pour établir si l'activité est de type "muscarinique" ou "nicotinique".

L'activité antagoniste éventuelle des produits est testée vis à vis de l'acétylcholine.

L'activité agoniste est exprimée en $pD_2$ (logarithme négatif de la dose qui produit 50% de l'effet maximum).

| Exemple | $pD_2$ |
|---|---|
| 2 | 5,97 |
| 6 | 5,71 |
| 8 | 5,23 |
| 10 | < 4 |
| Aérocoline, HBr | 6,48 |

**Activité diarrhéique**

Le test est réalisé sur des souris mâles ($CD_1$ Charles Rivers) pesant 25 à 30 g, à jeun depuis 6 heures. Le produit à 5% dans du méthocel est administré par voie orale, au moyen d'une sonde oesophagienne.

Des animaux témoins ne reçoivent que l'excipient.

Après traitement, les animaux sont mis séparément dans des cages dont le fond est recouvert de papier buvard et sont mis en observation pendant 30, 60, 120 et 180 minutes.

Les feuilles de papier absorbant sont changées après chaque observation.

La consistance des fèces est évaluée selon la méthode de Randall et Baruth (Arch. Int. Pharmacodyn. 220, 94, (1976)) en suivant l'échelle des valeurs suivantes.

O : consistance ferme,
1 : fèces légèrement molles avec ou sans auréole humide,
2 : fèces légèrement molles avec présence d'un cercle humide bien défini,
3 : fèces molles avec présence d'un grand cercle humide,
4 : fèces sans consistance avec présence d'un très grand cercle humide.

Pour chaque produit, on a noté la dose qui provoque une diarrhée chez 50% des animaux selon la

méthode de Miller et Tainter (Proc. Soc. Exp. Biol. Med. 57, 261, (1944)).

| Exemple | $DE_{50}$ en mg/kg |
|---|---|
| 2 | 3 |
| 6 | 15 |
| 10 | 10 |
| Aérocoline, HBr | 35 |

**Activité hypothermique,**

Le test est réalisé sur des souris mâles ($CD_1$ Charles Rivers) pesant 25 à 30 g, à jeun depuis 6 heures.

La température du corps est notée au moyen d'un thermocouple placé dans le rectum à environ 1,5 cm et relié à un enregistreur de température électrique.

Les produits sont administrés par voie orale ou sous-cutanée et les températures sont notées à l'instant 0 et 30 minutes, 1 heure, 2 heures et 2 heures et demie après traitement.

On évalue le degré d'hypothermie comme la différence entreles animaux traités et les témoins et on détermine la dose nécessaire pour réduire de 1 °C la température du corps.

| Exemple | Dose efficace (-1 °C) en mg/kg | |
|---|---|---|
| | V.O. | V.SC |
| 2 | 2,6 | 1,7 |
| 6 | 3,2 | 3,2 |
| 8 | 24 | 25 |
| 10 | 4,2 | 4,1 |
| Aérocoline, HBr | 194 | 3 |

**Revendications**

1.- Les composés de formule générale (I) :

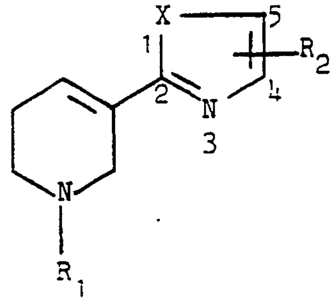

(I)

dans laquelle

- X représente un atome d'oxygène ou de soufre,
- $R_1$ représente un atome d'hydrogène, un radical alkyle linéaire, ramifié ou cyclisé renfermant jusqu'à 8 atomes de carbone, un radical alkényle ou alkynyle renfermant jusqu'à 6 atomes de carbone, un radical

hydroxyle, un radical OCOalc$_1$ dans lequel alc$_1$ représente un radical alkyle renfermant jusqu'à 6 atomes de carbone, un radical OCONHalc$_2$ dans lequel alc$_2$ représente un radical alkyle renfermant jusqu'à 6 atomes de carbone, un radical CO$_2$alc$_3$ dans lequel alc$_3$ représente un radical alkyle ou alkényle renfermant jusqu'à 6 atomes de carbone, un radical Oalc$_4$ dans lequel alc4 représente un radical alkyle renfermant jusqu'à 6 atomes de carbone, un radical OCOAr$_1$, OCONHAr$_2$ ou CO$_2$Ar$_3$ dans lequels Ar$_1$, Ar$_2$ ou Ar$_3$ représentent un radical aryle renfermant jusqu'à 14 atomes de carbone,

- R$_2$ représente un atome d'hydrogène, un radical alkyle linéaire, ramifié ou cyclisé, renfermant jusqu'à 6 atomes de carbone, un radical alkényle ou alkynyle renfermant jusqu'à 6 atomes de carbone, un radical alkoxyalkyle alc$'_1$Oalc$'_2$ dans lequel alc$'_1$ et alc$'_2$ représentent des radicaux alkyles renfermant jusqu'à 6 atomes de carbone, un radical hydroxyalkyle dans lequel le radical alkyle renferme jusqu'à 6 atomes de carbone ou un radical aryle renfermant jusqu'à 14 atomes de carbone, ainsi que leurs sels d'addition avec les acides organiques ou minéraux.

2.- Les composés de formule (I) tels que définis à la revendication 1, dans lesquels R$_1$ représente un radical hydroxy ainsi que leurs sels d'addition avec les acides organiques ou minéraux.

3.- Les composés de formule (I) tels que définis à la revendication 1, dans lesquels R$_1$ représente un atome d'hydrogène ainsi que leurs sels d'addition avec les acides organiques ou minéraux.

4.- Les composés de formule (I) tels que définis à la revendication 1, dans lesquels R$_1$ représente un radical alkyle linéaire, ramifié ou cyclique renfermant jusqu'à 6 atomes de carbone ainsi que leurs sels d'addition avec les acides organiques ou minéraux.

5.- Les composés de formule (I) tels que définis à la revendication 4 dans lesquels R$_1$ représente un radical méthyle ainsi que leurs sels d'addition avec les acides organiques ou minéraux.

6.- Les composés de formule (I) tels que définis à l'une quelconque des revendications 1 à 5, dans lesquels X représente un atome d'oxygène ainsi que leurs sels d'addition avec les acides organiques ou minéraux.

7.- Les composés de formule (I) tels que définis à l'une quelconque des revendications 1 à 6 dans lesquels R$_2$ représente un atome d'hydrogène ainsi que leurs sels d'addition avec les acides organiques ou minéraux.

8.- Les composés de formule (I) tels que définis à l'une quelconque des revendications 1 à 6, dans lesquels R$_2$ représente un radical alkyle linéaire, ramifié ou cyclique renfermant jusqu'à 6 atomes de carbone ainsi que leurs sels d'addition avec les acides organiques ou minéraux.

9.- Les composés de formule (I) tels que définis à la revendication 8 dans lesquels R$_2$ représente un radical méthyle ainsi que leurs sels d'addition avec les acides organiques ou minéraux.

10.- Les composés de formule (I) tels que dérinis à l'une quelconque des revendications 1 à 5 dans lesquels X représente un atome de soufre et R$_2$ représente un radical alkyle linéaire, ramifié ou cyclique renfermant jusqu'à 6 atomes de carbone ainsi que leurs sels d'addition avec les acides organiques ou minéraux.

11.- La 1-hydroxy 3-(oxazol-2-yl) 1,2,s,6-tétrahydropyridine et ses sels d'addition avec les acides organiques ou minéraux.

12.- La 3-(oxazol-2-yl) 1,2,5,6-tétrahydropyridine et ses sels d'addition avec les acides organiques ou minéraux.

13.- Les composés dont les noms suivent :

- la 3-(4-méthyloxazol-2-yl) 1,2,5,6-tétrahydropyridine,
- la 3-(5-méthyloxazol-2-yl) 1,2,5,6-tétrahydropyridine,

et leurs sels d'addition avec les acides organiques ou minéraux.

15.- A titre de médicaments, les composés de formule (I) tels que définis à l'une quelconque des revendications 1 à 10, ainsi que leurs sels d'addition avec les acides organiques ou minéraux pharmaceutiquement acceptables.

16.- A titre de médicaments, les composés de formule (I) tels que définis à l'une quelconque des revendications 11 à 13, ainsi que leurs sels d'addition avec les acides organiques ou minéraux pharmaceutiquement acceptables.

16.- Les compositions pharmaceutiques renfermant comme principe actif au moins un médicament défini à la revendication 14 ou 15.

17.- Procédé de préparation des composés de formule (I) tels que définis à l'une quelconque des revendications 1 à 13, caractérisé en ce que l'on soumet un composé de formule (II) :

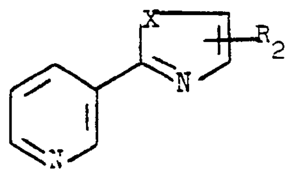

(II)

dans laquelle $R_2$ et X conservent leur signification précédente à l'action d'un composé de formule (III) :

$R'_1$-Hal (III)

dans laquelle $R'_1$ peut prendre toutes les valeurs données ci-dessus pour $R_1$ à l'exception de l'hydrogène, pour obtenir le composé de formule (IV) :

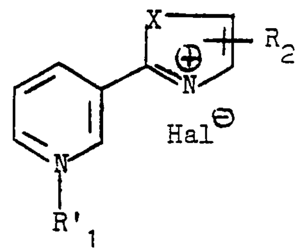

(IV)

que l'on soumet à l'action d'un agent de réduction pour obtenir le composé de formule ($I_A$) :

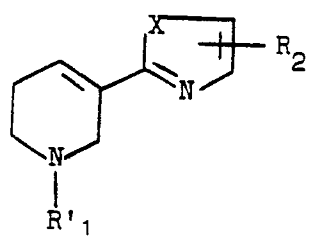

($I_A$)

que l'on salifie si désiré ou que l'on soumet le cas échéant à l'action d'un agent de clivage du groupe $R'_1$ pour obtenir le composé de formule ($I_B$) :

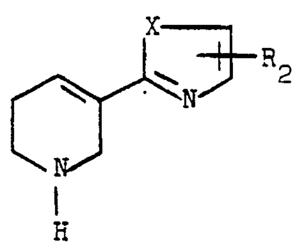

($I_B$)

que l'on salifie si désiré ou que l'on soumet, le cas échéant, à l'action d'un agent d'oxydation pour obtenir un composé de formule (I) dans laquelle $R_1$ représente un groupe $OR''_1$, dans lequel $R''_1$ représente un groupement protecteur du radical hydroxy que l'on salifie si désiré ou si désiré élimine le groupement protecteur pour obtenir un composé de formule (I) dans laquelle $R_1$ représente un radical hydroxy libre que, si désiré, l'on salifie.

18.- A titre de produit chimique nouveau, le composé de formule (II) défini à la revendication 17, dans lequel X est un atome d'oxygène et $R_2$ un radical méthyle en position 4.

Office européen des brevets

# RAPPORT DE RECHERCHE EUROPEENNE

Numero de la demande

EP 89 40 3667

## DOCUMENTS CONSIDERES COMME PERTINENTS

| Catégorie | Citation du document avec indication, en cas de besoin, des parties pertinentes | Revendication concernée | CLASSEMENT DE LA DEMANDE (Int. Cl.5) |
|---|---|---|---|
| X | SYNTHESIS août 1987, pages 693-696, Georg Thieme Verlag, Stuttgart, DE; A. DONDONI et al.: "Palladium-Catalyzed Coupling of Oxazol-2-yl- and 2-Oxazolin-2-yltrimethylstannanes with Aromatic Halides. A New Entry to 2-Aryl and 2-Heteroaryl Oxazoles and Oxazolines" * page 695; composé 8b * | 18 | C 07 D 413/04<br>C 07 D 417/04<br>A 61 K 31/44 |
| A | CHEMICAL ABSTRACTS tome 75, no. 23, 6 décembre 1971, page 339, colonne 2, abrégé no. 141029b, Columbus, Ohio, US; A. CHATTERIEE et al.: "Structure of aegelenine, the minor alkaloid of Aegle marmelos" & Indian Journal of Chemistry, 1971, tome 9, no. 8, pages 763-766 | 1,4-6 | |
| Y | EP-A-0 239 309 (MERCK SHARP & DOHME LTD.) * exemples 6-8; revendications 1,7-9 * | 1,3,6,7,14-16 | |
| Y | EP-A-0 261 763 (BEECHAM GROUP PLC) * exemples 1,5; revendications 1,12-15 * | 1,3,6,7,14-16 | |
| A | EP-A-0 244 018 (AKZO N.V.) * revendications 1,8-10 * | 1,15-17 | |
| P,X | EP-A-0 307 141 (MERCK SHARP & DOHME LTD.) * exemples 14-16,21,23,25; revendications 1-4,6-9 * | 1,3-10, 12-18 | |

-/-

**DOMAINES TECHNIQUES RECHERCHES (Int. Cl.5)**

C 07 D 413/00
C 07 D 417/00
A 61 K 31/00

Le présent rapport a été établi pour toutes les revendications

| Lieu de la recherche | Date d'achèvement de la recherche | Examinateur |
|---|---|---|
| BERLIN | 22-03-1990 | VAN AMSTERDAM L.J.P. |

**CATEGORIE DES DOCUMENTS CITES**

X : particulièrement pertinent à lui seul
Y : particulièrement pertinent en combinaison avec un autre document de la même catégorie
A : arrière-plan technologique
O : divulgation non-écrite
P : document intercalaire

T : théorie ou principe à la base de l'invention
E : document de brevet antérieur, mais publié à la date de dépôt ou après cette date
D : cité dans la demande
L : cité pour d'autres raisons

& : membre de la même famille, document correspondant

EPO FORM 1503 03.82 (P0402)

Office européen des brevets

# RAPPORT DE RECHERCHE EUROPEENNE

Numero de la demande

EP 89 40 3667

## DOCUMENTS CONSIDERES COMME PERTINENTS

| Catégorie | Citation du document avec indication, en cas de besoin, des parties pertinentes | Revendication concernée | CLASSEMENT DE LA DEMANDE (Int. Cl.5) |
|---|---|---|---|
| P,X | EP-A-0 296 721 (H. LUNDBECK A/S) * exemples 63,69-71,75,76; revendications 1-6 * ----- | 1,3-10, 12-18 | |

DOMAINES TECHNIQUES RECHERCHES (Int. Cl.5)

Le présent rapport a été établi pour toutes les revendications

| Lieu de la recherche | Date d'achèvement de la recherche | Examinateur |
|---|---|---|
| BERLIN | 22-03-1990 | VAN AMSTERDAM L.J.P. |

CATEGORIE DES DOCUMENTS CITES

X : particulièrement pertinent à lui seul
Y : particulièrement pertinent en combinaison avec un autre document de la même catégorie
A : arrière-plan technologique
O : divulgation non-écrite
P : document intercalaire

T : théorie ou principe à la base de l'invention
E : document de brevet antérieur, mais publié à la date de dépôt ou après cette date
D : cité dans la demande
L : cité pour d'autres raisons

& : membre de la même famille, document correspondant

EPO FORM 1503 03.82 (P0402)